# EUROPÄISCHE PATENTSCHRIFT

(11) **EP 2 059 494 B1**
(45) Veröffentlichungstag und Bekanntmachung des Hinweises auf die Patenterteilung: **24.11.2010**
(21) Anmeldenummer: 07801952.8
(22) Anmeldetag: 29.08.2007
(51) Int. Cl.: C07C 41/03, C07C 43/11, C07C 67/26, C07C 213/04, C08G 65/26

(54) **VERFAHREN ZUR HERSTELLUNG VON ALKYLENOXIDANLAGERUNGSPRODUKTEN**
METHOD FOR PRODUCING ALKYLENE OXIDE ADDITION PRODUCTS
PROCÉDÉ DE PRÉPARATION DE PRODUITS D'ADDITION D'OXYDES D'ALKYLÈNE

(30) Priorität: 07.09.2006 DE 102006041904
(43) Veröffentlichungstag der Anmeldung: 20.05.2009
(73) Patentinhaber: Cognis IP Management GmbH, 40589 Düsseldorf (DE)
(72) Erfinder: FRANZEN, Stefan, 59174 Kamen (DE); BÜTTGEN, Franz, 40721 Hilden (DE); MROZEK, Ingomar, 25348 Glückstadt (DE); GUTSCHE, Bernhard, 40724 Hilden (DE)
(86) Internationale Anmeldenummer: PCT/EP2007/007530
(87) Internationale Veröffentlichungsnummer: WO 2008/028586

(56) Entgegenhaltungen:
- EP-A- 1 586 372
- WO-A-00/51955
- "MICROREACTIORS FIND NEW NICHES" CHEMICAL ENGINEERING, ACCESS INTELLIGENCE ASSOCIATION, ROCKVILLE, MA, US, März 1997 (1997-03), Seiten 30-31,33, XP000197691 ISSN: 0009-2460

## Beschreibung

### Gebiet der Erfindung

Die Erfindung befindet sich auf dem Gebiet der nichtionischen Tenside und betrifft ein verbessertes Verfahren zur Herstellung von Alkylenoxidanlagerungsprodukten in der flüssigen Phase.

### Stand der Technik

Anlagerungsprodukte von Alkylenoxiden an Alkohole, Säuren oder Amine stellen wichtige technische Produkte dar, die vielfache Anwendung insbesondere als nichtionische Tenside finden.

Die Alkoxylierung wird üblicherweise diskontinuierlich, beispielsweise in Rührautoklaven oder Schlaufenreaktoren bei Temperaturen zwischen 80 und 130 °C durchgeführt; alternativ kann auch eine Dispersion der flüssigen Reaktionsmischung in eine alkylenoxidhaltige Gasphase erfolgen. Beispielhaft sei dazu auf einen Übersichtsartikel in Chem. Res. 25, 9482-9489 (2005**)** verwiesen. Typischerweise wird die Verbindung mit dem nucleophilen Zentrum - beispielsweise ein Alkohol, eine Carbonsäure, ein Ester oder ein Amin - zusammen mit dem Katalysator vorgelegt und dann die gewünschte Menge an Alkylenoxid aufgepresst, wobei sich in der Regel abhängig von der Temperatur ein Druck von bis zu 12 bar einstellt. Als Katalysatoren eignen sich basische Verbindungen, wie beispielsweise Alkalialkoholate, oder Lewis-Säuren, wobei letztere den Nachteil aufweisen, dass sie zur Bildung erheblicher Mengen unerwünschter Polyglykolether tendieren. Bei all diesen Prozessen sind eine flüssige Reaktionsmischung und eine alkylenoxidhaltige Gasphase beteiligt, was generisch mit dem Problem des unzureichenden Stofftransports an der Phasengrenzfläche und daher vermindertem Umsatz behaftet ist.

Die sicherheitstechnischen Probleme, die sich darüber hinaus durch Alkylenoxide in der Gasphase ergeben, sind z.B. in DE 10054462 B4 beschrieben. Durch die Anreicherung von Ethylen- oder Propylenoxid in der Gasphase besteht die Gefahr der Zersetzung. Daher wird die Konzentration des Alkylenoxids durch Inertgase niedrig gehalten. Gemäß der Lehre dieser Druckschrift wird die Alkoxylierung in einem Rohrreaktor durchgeführt, der als Plattenwärmeaustauscher ausgebildet ist. An mehreren Stellen im Reaktor wird Alkylenoxid eingespeist. Die Menge wird dabei gerade so gewählt, dass die Löslichkeit des Alkylenoxids im flüssigen Reaktionsgemisch nicht überschritten wird, um die lokale Entstehung einer Gasphase zu vermeiden.

Aus der EP 1586372 A1 (Goldschmidt) ist ein Verfahren zur Alkoxylierung verschiedenster Ausgangsstoffe in der flüssigen Phase bekannt, wobei die Reaktion in mikrostrukturierten Kapillarreaktoren erfolgt. Diese Mikroreaktoren werden erzeugt, indem man in einem Träger Scharen von Kanälen einätzt und diese auf dem Träger in einem Muster anordnet, bei dem die Strömungsrichtung ständig wechselt ("Zick-Zack-Muster").

In diesem Zusammenhang sei auch auf die internationale Patentanmeldung WO 02/009866 A1 (CPC) hingewiesen. Aus dieser Schrift ist ein Mikroreaktor u.a. auch zur Alkoxylierung von Fettalkoholen bekannt, bei dem die Reaktion zwischen zwei oder mehreren im wesentlichen planparallelen Platten oder Schichten stattfindet, wobei mindestens eine dieser Platten oder Schichten eine Fluidführungsplatte darstellt und diese so strukturiert und/oder angeordnet ist, dass der fluide Reaktionspartner nur aufgrund des Einflusses der Schwerkraft und/oder von Kapillarkräften in mindestens einem im wesentlichen ununterbrochenen Kapillarfaden entlang der Oberfläche oder Schicht fließt, und dabei mit dem gasförmigen Reaktionspartner in Kontakt trifft und reagiert. Es handelt sich hierbei jedoch um nichts anderes als einen miniaturisierten Fallfilmreaktor, bei dem die Reaktion an der Grenze von flüssiger und gasförmiger Phase stattfindet und die daher mit den bereits oben beschriebenen technischen Nachteilen behaftet ist.

Die Aufgabe der vorliegenden Erfindung hat somit darin bestanden, ein verbessertes Verfahren zur Herstellung von Alkylenoxidanlagerungsprodukten zur Verfügung zu stellen, welches einen intensiveren Stoffaustausch und damit höhere Umsätze ermöglicht und dabei auch höhere Alkylenoxidkonzentrationen zulässt, ohne dies durch verminderte Arbeitssicherheit zu erkaufen.

### Beschreibung der Erfindung

Gegenstand der Erfindung ist ein Verfahren zur Herstellung von Alkylenoxidanlagerungsprodukten durch Umsetzung von Verbindungen mit nucleophilem Zentrum mit Alkylenoxiden in einem Reaktor mit hoher innerer Oberfläche, welches sich dadurch auszeichnet, dass man
(i) die Umsetzung in Mikrorohrbündelreaktoren durchführt, welche aus 10 bis 100 einzelnen Kapillaren mit einer Länge von 5 bis 1000 cm und einem Durchmesser von 0,25 bis 15 mm bestehen, und
(ii) die Einspeisung der Einsatzstoffe über eine Misch- und Dosiervorrichtung vornimmt, in der das gasförmige oder verflüssigte Alkylenoxid im flüssigen Feedstrom gelöst wird, so dass die Alkoxylierung ausschließlich in der Flüssigphase stattfindet.

Überraschenderweise wurde gefunden, dass die Alkoxylierung in der flüssigen Phase unter Einsatz von speziellen Mikroreaktionsrohrbündeln aus dem Stand der Technik bekannten Nachteile überwindet und nun insbesondere auch der Einsatz von höheren Alkylenoxidkonzentrationen möglich ist, ohne dass es zu sicherheitstechnischen Problemen kommt. Gleichzeitig wird das Problem des unzureichenden Kontaktes zwischen Dampf- und Flüssigphase vermieden, so dass das Verfahren auch zu höheren Umsätzen führt.

### Verbindungen mit nucleophilen Molekülgruppen

Die Auswahl des Reaktionspartners für die nachfolgende Alkoxylierung ist an sich unkritisch. Bedingung ist lediglich, dass es sich um eine Verbindung mit einem nucleophilen Zentrum, vorzugsweise mit einem aziden Wasserstoffatom handelt. Für diesen Zweck kommen insbesondere Alkohole der Formel (I) in Frage,

R¹OH (I)

in der R¹ für einen linearen oder verzweigten Kohlenwasserstoffrest mit 1 bis 22, vorzugsweise 8 bis 18 Kohlenstoffatomen und 0 oder 1 bis 3 Doppelbindungen steht. Typische Beispiele sind neben den niederen aliphatischen Alkoholen Methanol, Ethanol sowie den isomeren Butanolen und Pentanolen die Fettalkohole, nämlich Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Eine weitere Gruppe von Verbindungen, die sich als Ausgangsstoffe für die Alkoxylierung eignen, bilden die Carbonsäuren der Formel (II),

R²CO-OH (II)

in der R²CO für einen linearen oder verzweigten Acylrest mit 1 bis 22 Kohlenstoffatomen und 0 oder 1 bis 3 Doppelbindungen steht. Typische Beispiele sind vor allem die Fettsäuren, nämlich Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure. Selbstverständlich können auch funktionalisierte Carbonsäuren, z.B. Hydroxycarbonsäuren wie die Rizinolsäure oder die Zitronensäure oder Dicarbonsäuren wie die Adipinsäure alkoxyliert werden. Anstelle der Säuren können auch die entsprechenden Ester mit C₁-C₂₂ Alkoholen oder Glycerin eingesetzt werden; hier findet dann eine Insertion in die Estergruppe statt.

Anstelle der Carbonsäuren können deren Ester mit Alkoholen mit 1 bis 22 und vorzugsweise 1 bis 4 Kohlenstoffatomen oder Polyolen, speziell Glycerin, Trimethylolpropan oder Pentaerythrit eingesetzt werden. Neben den entsprechenden Carbonsäuremethylester sind die Glyceride und darunter wieder die Partialglyceride bevorzugt. Sofern es sich um Vollester handelt, erfolgt ein Einschub der Alkylenoxidgruppen in die Carbonylesterbindung. Schließlich eignen sich als weitere Gruppe von Verbindungen auch Amine der Formel (III) als Ausgangsstoffe,

R³-NH-R⁴ (III)

in der R³ und R⁴ unabhängig voneinander für Wasserstoff, Alkylgruppen mit 1 bis 18 Kohlenstoffatomen oder Hydroxyalkylgruppen mit 1 bis 4 Kohlenstoffatomen stehen. Typische Beispiele sind Methylamin, Dimethylamin, Ethylamin, Diethylamin, Methylethylamin sowie die verschiedenen analog aufgebauten Propyl-, Butyl-, Pentyl- und Fettamine.

### Mikrorohrbündelreaktoren

Unter dem Begriff "strukturierter Reaktor" ist im Allgemeinen eine Anordnung von Reaktionskanälen zu verstehen, die einzeln, in Modulen oder auch alle gemeinsam betrieben werden können und sich in einer Matrix befinden, die zur Stabilisierung, Sicherung, Heizung oder Kühlung dient. Im Sinne der Erfindung werden unter strukturierten Reaktoren ausschließlich Mikrorohrbündelreaktoren verstanden, welche typischerweise aus 10 bis 100 einzelnen Kapillaren mit einer Länge von 5 bis 1000 cm und einem Durchmesser von 0,25 bis 15 mm bestehen können. Die Einspeisung der Einsatzstoffe erfolgt über eine Misch- und Dosiervorrichtung, in der das gasförmige, vorzugsweise aber verflüssigte Alkylenoxid im flüssigen Feedstrom gelöst wird, so dass die Alkoxylierung ausschließlich in der Flüssigphase stattfindet. Die Temperierung der Reaktoren kann beispielsweise mit Hilfe eines Druckwasserkreislaufs, oder durch eine Kombination aus Kühlwasser- und Dampfkreislauf, Thermalöl oder Siedekühlung erfolgen.

### Alkoxylierung

Die Alkoxylierung findet vorzugsweise in Gegenwart von Katalysatoren statt, die homogener oder heterogener Natur sein können. Bei Einsatz von homogenen Katalysatoren empfiehlt es sich, diese in den Verbindungen mit nucleophilem Zentrum, also beispielsweise in einem Alkohol, zu lösen bzw. zu dispergieren und so dem erfindungsgemäßen Reaktor zuzuführen; dies ist insbesondere im Fall der Mikrorohrbündelreaktoren empfehlenswert. Als geeignete homogene Katalysatoren seien beispielhaft Alkalihydroxide oder Alkalialkoholate, speziell Kaliumhydroxid, Kalium-tert-butylat und insbesondere Natriummethylat genannt. Werden heterogene Katalysatoren eingesetzt, so werden diese vorzugsweise durch Beschichtung der Kanäle der Mikroreaktionssystems oder aber als Monolithstruktur bzw. als Festbett eingesetzt. Vorzugsweise weisen diese Schichten dann im Mittel eine Stärke von 50 bis 2.000 und insbesondere 100 bis 1.000 nm auf. Ein bevorzugtes Beispiel für einen geeigneten Katalysator, den man beispielsweise durch Imprägnieren und nachfolgendes Calcinieren aufbringt, ist das Hydrotalcit. In der Regel setzt man das als Alkylenoxide Ethylen- und/oder Propylenoxid ein, wobei das molare Verhältnis zwischen den Alkylenoxiden und den Verbindungen mit nucleophilem Zentrum 1:1 bis 200:1, vorzugsweise 5:1 bis 50:1 und insbesondere 8:1 bis 20:1 betragen kann. Die Reaktionstemperatur kann dabei in Abhängigkeit vom Einsatzmaterial zwischen 50 und 200 °C variieren und liegt vorzugsweise bei 100 bis 140 °C, während der Druck 1 bis 100, vorzugsweise 4 bis 50 und insbesondere 5 bis 30 bar betragen kann. Dabei ist zu beachten, dass der Betriebsdruck im Reaktor jederzeit - also auch bei maximaler Betriebstemperatur - höher als der Dampfdruck des Alkylenoxids ist, so dass sich keine Alkylenoxiddampfphase bilden kann und die Alkoxylierung tatsächlich ausschließlich in der flüssigen Phase stattfindet.

### Beispiele

### Beispiel 1

Ein technisches C_{12/14}-Kokosfettalkoholgemisch (Lorol^{®} Spezial, Cognis GmbH) wurde im Durchstromerhitzer auf 130 °C vorgeheizt. In den Rohstoffstrom wurde dann eine 45 Gew.-%ige wässrige Kaliumhydroxidlösung eindosiert, so dass sich eine KOH-Konzentration von ca. 0,1 Gew.-% einstellte. Die so erhaltene Mischung wurde in einem kontinuierlich arbeitenden Mikro-Fallfilmreaktor bei einer Temperatur von ca. 130 °C und einem verminderten Druck von ca. 200 mbar vom Wasser befreit. Sowohl der getrocknete Feedstrom als auch das Ethylenoxid wurden dann mit Hilfe von zwei Pumpen auf einen Druck von 30 bar verdichtet und gemeinsam über einen Flüssigkeitsverteiler in einen Mikrorohrbündelreaktor bestehend aus 50 Edelstahlkapillaren mit einer Länge von 25 cm und einem Durchmesser von 1 mm eindosiert. Die bei der Anlagerung des Ethylenoxids and den Alkohol freiwerdende Wärme führt zu einem Anstieg der Temperatur im Reaktor auf 165 bis 180 °C. Aus diesem Grunde wurde der Rohrbündelreaktor mit einem Druckwasserkreislauf gekühlt, mit dessen Hilfe sichergestellt wurde, dass das die Rohrbündel verlassende Alkoxylierungsprodukt nur noch eine Temperatur von 80 °C aufwies. Die über den Kühlwasserkreislauf abgeführte Wärme wurde genutzt, um weiteren Fettalkohol auf die Temperatur von 130 °C vorzuwärmen.

## Patentansprüche

1. Verfahren zur Herstellung von Alkylenoxidanlagerungsprodukten durch Umsetzung von Verbindungen mit nucleophilem Zentrum mit Alkylenoxiden in einem Reaktor mit hoher innerer Oberfläche, **dadurch gekennzeichnet, dass** man
(i) die Umsetzung in Mikrorohrbündelreaktoren durchführt, welche aus 10 bis 100 einzelnen Kapillaren mit einer Länge von 5 bis 1000 cm und einem Durchmesser von 0,25 bis 15 mm bestehen, und
(ii) die Einspeisung der Einsatzstoffe über eine Misch- und Dosiervorrichtung vornimmt, in der das gasförmige oder verflüssigte Alkylenoxid im flüssigen Feedstrom gelöst wird, so dass die Alkoxylierung ausschließlich in der Flüssigphase stattfindet.

2. Verfahren nach Anspruch 1, **dadurch gekennzeichnet, dass** man als Verbindung mit nucleophilem Zentrum Alkohole der Formel (I) einsetzt,
R¹OH (I)
in der R¹ für einen linearen oder verzweigten Kohlenwasserstoffrest mit 1 bis 22 Kohlenstoffatomen und 0 oder 1 bis 3 Doppelbindungen steht.

3. Verfahren nach Anspruch 1, **dadurch gekennzeichnet, dass** man als Verbindung mit nucleophilem Zentrum Carbonsäuren der Formel (II) einsetzt,
R²CO-OH (II)
in der R²CO für einen linearen oder verzweigten Acylrest mit 1 bis 22 Kohlenstoffatomen und 0 oder 1 bis 3 Doppelbindungen steht.

4. Verfahren nach Anspruch 1 und 3, **dadurch gekennzeichnet, dass** man als Verbindung mit nucleophilem Zentrum Voll- oder Partialester der Carbonsäuren der Formel (II) mit aliphatischen Alkoholen mit 1 bis 22 Kohlenstoffatomen oder Polyolen einsetzt.

5. Verfahren nach Anspruch 1, **dadurch gekennzeichnet, dass** man als Verbindung mit nucleophilem Zentrum Amine der Formel (III) einsetzt,
R³-NH-R⁴ (III)
in der R³ und R⁴ unabhängig voneinander für Wasserstoff, Alkylgruppen mit 1 bis 18 Kohlenstoffatomen oder Hydroxyalkylgruppen mit 1 bis 4 Kohlenstoffatomen stehen.

6. Verfahren nach mindestens einem der Ansprüche 1 bis 5, **dadurch gekennzeichnet, dass** man die Alkoxylierung in Gegenwart von homogenen oder heterogenen Katalysatoren durchführt.

7. Verfahren nach Anspruch 6, **dadurch gekennzeichnet, dass** man die homogenen Katalysatoren in den Verbindungen mit nucleophilem Zentrum löst bzw. dispergiert.

8. Verfahren nach Anspruch 6, **dadurch gekennzeichnet, dass** man die Mikrorohrbündelreaktoren mit den heterogenen Alkoxylierungskatalysatoren beschichtet oder als Monolithstruktur bzw. Festbett auslegt.

9. Verfahren nach mindestens einem der Ansprüche 1 bis 8, **dadurch gekennzeichnet, dass** man als Alkylenoxide Ethylenoxid, Propylenoxid oder deren Gemische einsetzt.

10. Verfahren nach mindestens einem der Ansprüche 1 bis 9, **dadurch gekennzeichnet, dass** man die Alkylenoxide und die Verbindungen mit nucleophilem Zentrum im molaren Verhältnis von 1:1 bis 200:1 umsetzt.

11. Verfahren nach mindestens einem der Ansprüche 1 bis 10, **dadurch gekennzeichnet, dass** man die Alkoxylierung bei Temperaturen im Bereich von 50 bis 200 °C durchführt.

12. Verfahren nach mindestens einem der Ansprüche 1 bis 11, **dadurch gekennzeichnet, dass** man die Alkoxylierung bei Drücken im Bereich von 1 bis 100 bar durchführt.

13. Verfahren nach mindestens einem der Ansprüche 1 bis 12, **dadurch gekennzeichnet, dass** der Betriebsdruck jederzeit höher als der Dampfdruck des Alkylenoxids ist.

## Claims

1. Process for preparing alkylene oxide addition products by reacting compounds having a nucleophilic site with alkylene oxides in a reactor with high internal surface area, **characterized in that**
(i) the reaction is performed in micro shell and tube reactors which consist of 10 to 100 individual capillaries with a length of 5 to 1000 cm and a diameter of 0.25 to 15 mm, and
(ii) feeding in the feedstocks by means of a mixing and metering apparatus in which the gaseous or liquefied alkylene oxide is dissolved in the liquid feed stream, such that the alkoxylation takes place exclusively in the liquid phase.

2. Process according to Claim 1, **characterized in that** the compounds having nucleophilic sites used are alcohols of the formula (I)
R¹OH (I)
in which R¹ is a linear or branched hydrocarbon radical having 1 to 22 carbon atoms and 0 or 1 to 3 double bonds.

3. Process according to Claim 1, **characterized in that** the compounds having nucleophilic sites used are carboxylic acids of the formula (II)
R²CO-OH (II)
in which R²CO is a linear or branched acyl radical having 1 to 22 carbon atoms and 0 or 1 to 3 double bonds.

4. Process according to Claims 1 and 3, **characterized in that** the compounds having nucleophilic sites used are full or partial esters of the carboxylic acids of the formula (II) with aliphatic alcohols having 1 to 22 carbon atoms or polyols.

5. Process according to Claim 1, **characterized in that** the compounds having nucleophilic sites used are amines of the formula (III)
R³-NH-R⁴ (III)
in which R³ and R⁴ are each independently hydrogen, alkyl groups having 1 to 18 carbon atoms or hydroxyalkyl groups having 1 to 4 carbon atoms.

6. Process according to at least one of Claims 1 to 5, **characterized in that** the alkoxylation is performed in the presence of homogeneous or heterogeneous catalysts.

7. Process according to Claim 6, **characterized in that** the homogeneous catalysts are dissolved or dispersed in the compounds having nucleophilic sites.

8. Process according to Claim 6, **characterized in that** the micro shell and tube reactors are coated with the heterogeneous alkoxylation catalysts or are designed as a monolith structure or fixed bed.

9. Process according to at least one of Claims 1 to 8, **characterized in that** the alkylene oxides used are ethylene oxide, propylene oxide or mixtures thereof.

10. Process according to at least one of Claims 1 to 9, **characterized in that** the alkylene oxides and the compounds having nucleophilic sites are reacted in a molar ratio of 1:1 to 200:1.

11. Process according to at least one of Claims 1 to 10, **characterized in that** the alkoxylation is performed at temperatures in the range from 50 to 200°C.

12. Process according to at least one of Claims 1 to 11, **characterized in that** the alkoxylation is performed at pressures in the range from 1 to 100 bar.

13. Process according to at least one of Claims 1 to 12, **characterized in that** the operating pressure is always higher than the vapour pressure of the alkylene oxide.

## Revendications

1. Procédé pour la préparation de produits d'addition d'oxyde d'alkylène par transformation de composés avec un centre nucléophile avec des oxydes d'alkylène dans un réacteur présentant une grande surface interne, **caractérisé en ce qu'**on
(i) réalise la transformation dans des réacteurs à faisceau microtubulaire, qui sont constitués par 10 à 100 capillaires individuels présentant une longueur de 5 à 1000 cm et un diamètre de 0,25 à 15 mm, et
(ii) réalise l'injection des substances de départ via un dispositif de mélange et de dosage, dans lequel l'oxyde d'alkylène gazeux ou liquéfié est dissous dans le flux alimenté liquide de manière telle que l'alcoxylation se produise exclusivement dans la phase liquide.

2. Procédé selon la revendication 1, **caractérisé en ce qu'**on utilise comme composé présentant un centre nucléophile des alcools de formule (I),
R¹OH (I)
dans laquelle R¹ représente un radical hydrocarboné linéaire ou ramifié comprenant 1 à 22 atomes de carbone et 0 ou 1 à 3 doubles liaisons.

3. Procédé selon la revendication 1, **caractérisé en ce qu'**on utilise comme composé présentant un centre nucléophile des acides carboxyliques de formule (II),
R²CO-OH (II)
dans laquelle R²CO représente un radical acyle linéaire ou ramifié comprenant 1 à 22 atomes de carbone et 0 ou 1 à 3 doubles liaisons.

4. Procédé selon les revendications 1 et 3, **caractérisé en ce qu'**on utilise comme composé présentant un centre nucléophile des esters complets ou partiels des acides carboxyliques de formule (II) avec des alcools aliphatiques comprenant 1 à 22 atomes de carbone ou des polyols.

5. Procédé selon la revendication 1, **caractérisé en ce qu'**on utilise comme composé présentant un centre nucléophile des amines de formule (III),
R³-NH-R⁴ (III)
dans laquelle R³ et R⁴ représentent, indépendamment l'un de l'autre, hydrogène, des groupes alkyle comprenant 1 à 18 atomes de carbone, ou des groupes hydroxyalkyle comprenant 1 à 4 atomes de carbone.

6. Procédé selon au moins l'une quelconque des revendications 1 à 5, **caractérisé en ce qu'**on réalise l'alcoxylation en présence de catalyseurs homogènes ou hétérogènes.

7. Procédé selon la revendication 6, **caractérisé en ce qu'**on dissout ou disperse les catalyseurs homogènes dans les composés présentant un centre nucléophile.

8. Procédé selon la revendication 6, **caractérisé en ce qu'**on recouvre les réacteurs à faisceau microtubulaire avec les catalyseurs d'alcoxylation hétérogènes ou qu'on les agence comme structure monolithique ou lit fixe.

9. Procédé selon au moins l'une quelconque des revendications 1 à 8, **caractérisé en ce qu'**on utilise comme oxydes d'alkylène l'oxyde d'éthylène, l'oxyde de propylène ou leurs mélanges.

10. Procédé selon au moins l'une quelconque des revendications 1 à 9, **caractérisé en ce qu'**on transforme les oxydes d'alkylène et les composés présentant un centre nucléophile dans un rapport molaire de 1:1 à 200:1.

11. Procédé selon au moins l'une quelconque des revendications 1 à 10, **caractérisé en ce qu'**on réalise l'alcoxylation à des températures dans la plage de 50°C à 200°C.

12. Procédé selon au moins l'une quelconque des revendications 1 à 11, **caractérisé en ce qu'**on réalise l'alcoxylation à des pressions dans la plage de 1 à 100 bars.

13. Procédé selon au moins l'une quelconque des revendications 1 à 12, **caractérisé en ce que** la pression d'exploitation est à tout moment supérieure à la tension de vapeur de l'oxyde d'alkylène.
